# EUROPÄISCHE PATENTSCHRIFT

(11) **EP 1 372 786 B1**
(45) Veröffentlichungstag und Bekanntmachung des Hinweises auf die Patenterteilung: **11.05.2011**
(21) Anmeldenummer: 02722273.6
(22) Anmeldetag: 27.03.2002
(51) Int. Cl.: A61P 39/00, A61K 31/662, C12N 5/077

(54) **ZYTOPROTEKTION DURCH PHOSPHOTYROSIN**
CYTOPROTECTION BY PHOSPHOTYROSINE
CYTOPROTECTION AU MOYEN DE PHOSPHOTYROSINE

(30) Priorität: 03.04.2001 DE 10117834
(43) Veröffentlichungstag der Anmeldung: 02.01.2004
(73) Patentinhaber: Eberhard-Karls-Universität Tübingen Universitätsklinikum, 72076 Tübingen (DE)
(72) Erfinder: DITTMANN, Klaus, 72070 Tübingen (DE); MAYER, Claus, 72072 Tübingen (DE); RODEMANN, Peter, 70192 Stuttgart (DE)
(74) Vertreter: Otten, Hajo
(86) Internationale Anmeldenummer: PCT/EP2002/003403
(87) Internationale Veröffentlichungsnummer: WO 2002/081028

(56) Entgegenhaltungen:
- DE-A- 19 720 339
- MISHRA, SHRIKANT ET AL: "Association of inhibition of cell growth by O-phospho-L-tyrosine with decreased tyrosine phosphorylation" CANCER LETTERS (SHANNON, IRELAND) (1996), 102(1,2), 65-71 , XP002217214
- MISHRA SHRIKANT ET AL: "O-Phospho-L-tyrosine inhibits cellular growth by activating protein tyrosine phosphatases." CANCER RESEARCH, Bd. 53, Nr. 3, 1993, Seiten 557-563, XP002218110 ISSN: 0008-5472 in der Anmeldung erwähnt
- DITTMANN K H ET AL: "O-phospho-L-tyrosine protects TP53 wild-type cells against ionizing radiation." INTERNATIONAL JOURNAL OF CANCER. JOURNAL INTERNATIONAL DU CANCER. UNITED STATES 2001, Bd. 96 Suppl, 2001, Seiten 1-6, XP001113018 ISSN: 0020-7136

## Beschreibung

Die vorliegende Erfindung beschäftigt sich mit dem Schutz von biologischem Material gegenüber zellschädigenden Faktoren.

Es ist bekannt, daß biologisches Material, wie z.B. tierische oder menschliche Zellen, Gewebe oder Organismen durch eine Vielzahl verschiedenster Faktoren in seiner Integrität geschädigt werden kann. Solche Faktoren können z.B. energiereiche bzw. ionisierende Strahlung, wie z.B. radioaktive Strahlung, Röntgenstrahlung, Höhenstrahlung etc. oder ultraviolettes Licht (UV-Licht), sein. Ionisierende Strahlung wird einerseits zu den Umweltnoxen gezählt, sie kann andererseits aber auch nutzbar in der Medizin eingesetzt werden. In diesem Zusammenhang sei die Röntgendiagnostik, Nuklearmedizin oder Strahlentherapie genannt. Diese energiereiche Strahlung zeichnet sich dadurch aus, daß sie die Fähigkeit hat, Moleküle zu ionisieren.

Weitere wichtige zellschädigende Faktoren sind chemische Agenzien, z.B. in Form von Umweltgiften, aber auch solche, die im Rahmen von medizinischen Behandlungen als Therapeutika, z.B. Zytostatika, verwendet werden. Bei letzteren handelt es sich um eine chemisch heterogene Gruppe zytotoxischer pharmakologischer Substanzen, die die Zellteilung funktionell aktiver Zellen durch unterschiedliche Beeinflussung ihres Stoffwechsels verhindern oder erheblich verzögern. Zytostatika werden hauptsächlich in der Tumortherapie eingesetzt, wobei der Ansatzpunkt für die Wirksamkeit die gegenüber normalen Zellen gesteigerte Zellteilungsrate der Tumorzellen ist. Man kennt verschiedene Gruppen von Zytostatika: Alkylanzien (z.B. Cisplatin); Antimetabolite (z.B. Folsäureantagonisten); Mitosehemmstoffe; Antibiotika (z.B. Bleomycin); Enzyme (z.B. L-Asparaginase); und andere.

Diese Faktoren können auf allen Ebenen biologischer Strukturierung Schäden hervorrufen: so zeigen sich auf ein Einwirken dieser Faktoren Reaktionen auf Molekül- bzw. Makromolekül- (z.B. Nukleinsäure-)ebene, auf zellulärer Ebene, Gewebsreaktionen oder Reaktionen des Gesamtorganismus.

Durch energiereiche oder ultraviolette Strahlung hervorgerufene Schäden sind beispielsweise die Veränderung von DNA, also die Mutagenese, die zu Tumorentstehung führen kann, sowie die Degeneration, Atrophie, Fibrosierung oder Nekrose von solchen Geweben, die hoher Strahlung ausgesetzt sind.

So wird beispielsweise die Entstehung des malignen Melanoms durch hohe Sonnenbelastung der Haut gefördert.

Wie eingangs erwähnt, ist der menschliche Organismus nicht nur bei starker Sonnenlichtexposition, sondern bei entsprechender medizinischer Indikation auch während der Röntgendiagnostik oder im Falle einer Strahlentherapie im Zusammenhang mit Tumorerkrankungen mit besonders hohen Strahlungsintensitäten konfrontiert. Besonders gefährdet sind in diesem Zusammenhang auch Radiologen, Zahnärzte, Unfallchirurgen, Radiologisch-Technische Assistenten und Arbeiter in Röntgenröhrenfabriken.

Die hinsichtlich Zellschädigung als besonders bedeutsam einzustufenden, oben genannten Zytostatika führen vor allem deshalb bei ihrer Verwendung im menschlichen Organismus zu Schäden, weil die Unterschiede zwischen normalen und Tumorzellen bezüglich der Zellteilungsrate für einen selektiven tumorspezifischen Angriffspunkt nicht ausreichen. Daher resultieren die unerwünschten Nebenwirkungen von Zytostatika vor allem aus der generellen Regenerationshemmung rasch proliferierender Gewebe. Besonders betroffen sind die Bildung der Blutzellen, die Epithelien der Schleimhäute, deren Regenerationshemmung zu gastrointestinalen Störungen führt, sowie Haut und Hautanhangsgebilde, deren Regenerationshemmung zu Haarausfall führt.

Aus der DE 197 20 339 A1 ist die Verwendung von Phosphotyrosin zur Herstellung einer dermatologischen Zusammensetzung zur Verhinderung und Behandlung der durch UV-Strahlung hervorgerufenen Hautbräunung. Die Autoren geben jedoch keinerlei Informationen darüber, wie eine entsprechende Verhinderung oder Behandlung von Hautschädigungen zu erfolgen hat, die durch UV-Strahlung hervorgerufen wurden.

Vor diesem Hintergrund ist es Aufgabe der vorliegenden Erfindung, einen Schutz von biologischem Material vor den eingangs genannten Pathologien, die ausgelöst werden können durch zellschädigende Faktoren, wie Strahlung, vorzugsweise ionisierende Strahlung oder ultraviolettes Licht, sowie Chemikalien, vorzugsweise Zytostatika, bereitzustellen.

Diese Aufgabe wird erfindungsgemäß durch die Verwendung von Phosphotyrosin zur Herstellung einer pharmazeutischen Zusammensetzung zu den genannten Zwecken gelöst.

Phosphotyrosin (im folgenden auch P-Tyr) ist eine modifizierte, von der aromatischen Aminosäure Tyrosin abgeleitete Aminosäure, bei der die Hydroxylgruppe des Phenyls der Seitenkette phosphoryliert wurde. Phosphotyrosin ist in einer Vielzahl von Lehrbüchern der Biochemie, Molekularbiologie und Proteinchemie beschrieben.

Die Erfinder haben überraschenderweise erkannt, daß diese phosphorylierte Aminosäure biologisches Material vor zellschädigenden Faktoren schützt.

Dabei wird im Sinne der Erfindung unter Phosphotyrosin sowohl O-Phospho-L-Tyrosin (L-3-[4-Hydroxyphenyl]alanin-4'-Phosphat) als auch O-Phospho-D-Tyrosin (D-3-[4-Hydroxyphenyl]alanin-4'-Phosphat) und O-Phospho-DL-Tyrosin (DL-3-[4-Hydroxyphenyl]alanin-4'-Phosphat) verstanden.

In diesem Zusammenhang ist unter biologischem Material jegliche biologische strukturierte Einheit wie z. B. eine Zelle (in Kultur oder im Gewebeverband), Gewebe, Organe, Organismen etc. zu verstehen.

Unter zellschädigenden Faktoren im Sinne der Erfindung sind sich negativ auf die Integrität und/oder Viabilität von biologischem Material auswirkenden Einflüsse zu verstehen, nämlich ionisierende Strahlung (radioaktive Strahlung, Röntgenstrahlung, Höhenstrahlung etc.), ultraviolettes Licht, Chemikalien jeder Art, insbesondere Zytostatika (beispielsweise alkylierende Verbindungen wie Cisplatin).

Unter der Verwendung von Phosphotyrosin ist zu verstehen, daß diese modifizierte Aminosäure entweder in einer geeigneten, z.B. galenischen Aufarbeitung mit biologischem Material in Kontakt gebracht wird, z.B. auf einen Organismus aufgebracht oder in diesen eingebracht wird, oder *in vitro* in irgendeiner Form mit biologischem Material, z.B. isolierten Zellen, Geweben oder Organen in Kontakt gebracht wird. Dabei kann das In-Kontakt-Bringen mit dem Phosphotyrosin vor, während oder nach der Exposition an zellschädigenden Faktoren erfolgen.

Schutz von biologischem Material bedeutet im Sinne der Erfindung, daß die durch die genannten zellschädigenden Faktoren hervorgerufenen Schädigungen durch Phosphotyrosin reduziert oder verhindert werden.

Ein Vorteil bei der erfindungsgemäßen Verwendung von Phosphotyrosin ist darin zu sehen, daß Phosphotyrosin in großen Mengen kostengünstig herstellbar bzw. erhältlich ist. Hierfür ist keine Peptidsynthese oder aufwendige Proteinreinigung notwendig.

Außerdem ist Phosphotyrosin im Vergleich zu Peptiden oder Proteinen deutlich resistenter gegenüber Degradation. Damit ist auch eine galenische Aufbereitung wesentlich einfacher und stabiler.

Aufgrund der geringen molekularen Größe des Phosphotyrosins ist dessen mögliche Aufnahme in die zu behandelnden Zellen erleichtert, was zu einer guten zytoprotektiven Wirksamkeit führt. Auch die Gefahr von immunologischen Reaktionen im Falle des Einbringens oder Aufbringens von Phosphotyrosin in bzw. auf einen Organismus ist aufgrund der geringen Größe nicht zu erwarten.

Die zytoprotektiven Eigenschaften von Phosphotyrosin sind auch deshalb überraschend, weil Phosphotyrosin bisher ganz andere Aktivitäten zugeschrieben wurden. So haben beispielsweise Shrikant Mishra und Anne W. Hamburger gezeigt, daß Phosphotyrosin das Wachstum von menschlichen Brust- und Nierenkarzinomzellen hemmt ("O-phospo-L-tyrosine inhibits cellular growth by activating protein tyrosine phosphatases", Cancer Research 53, S. 557-563, 1993), und daß diese Wachstumshemmung P-Tyrdosisabhängig erfolgt ("Exogenous phosphotyrosine modulates epidermal growth factor receptor tyrosine phosphorylation", Carcinogenesis 14, S. 269-273, 1993). Die Hemmung des Zellwachstums durch Phosphotyrosin wurde von dieser Arbeitsgruppe für zwei weitere Tumorzellinien gezeigt, für eine Leberkarzinomzellinie und für src-transformierte NIH 3T3-Zellen ("Association of inhibition of all growth by O-phospho-L-tyrosine with decreased phosphorylation", Cancer Letters 102, S. 65-71, 1996). Ein Schutz gesunder Zellen durch Behandlung mit Phosphotyrosin wird von den Autoren nicht beschrieben.

Die Erfinder der vorliegenden Anmeldung konnten eine inhibierende Wirkung durch eine alleinige Phosphotyrosingabe auf das Wachstum von Tumorzellen nicht bestätigen. Sie konnten hingegen überraschenderweise ein verstärktes Absterben von mit Phosphotyrosin vorbehandelten Tumorzellen nach Bestrahlung mit ionisierenden Strahlen im Vergleich zu nicht vorbehandelten Tumorzellen feststellen.

Weiter stellte sich heraus, daß anders als bei Tumorzellen mit Phosphotyrosin vorbehandelte Normalzellen sowohl nach Bestrahlung mit ionisierenden Strahlen als auch nach Behandlung mit Cisplatin eine signifikant höhere Überlebensrate gegenüber nicht mit Phosphotyrosin vorbehandelten Normalzellen zeigen.

Diese Selektivität der zytoprotektiven Eigenschaft von Phosphotyrosin für Normalzellen, also für gesunde Nicht-Tumorzellen war angesichts der im Stand der Technik bekannten, in einem völlig verschiedenen Zusammenhang beschriebenen Eigenschaften von Phosphotyrosin nicht zu erwarten.

Somit wird die der Erfindung zugrunde liegende Aufgabe vollkommen gelöst.

Das Phosphotyrosin wird zum Schutz von biologischem Material gemäß Anspruchs 1 gegenüber Strahlung, vorzugsweise ionisierender Strahlung und/oder ultraviolettem Licht verwendet.

Dies hat den besonderen Vorteil, daß damit eine Verwendung bereitgestellt wird, die Schutz vor besonders bedeutsamen zellschädigenden Faktoren bietet, denen auch nahezu jeder menschliche Organismus, zumindest partiell, ausgesetzt ist.

Weiterhin ist es bevorzugt, Phosphotyrosin zum Schutz von bio-logischem Material gemäß Anspruchs 1 gegenüber Chemikalien, vorzugsweise Zytostatika zu verwenden.

Wie eingangs erwähnt, spielen Chemikalien, insbesondere Zytostatika als zellschädigende Faktoren eine bedeutende Rolle.

Diese bevorzugte erfindungsgemäße Verwendung von Phosphotyrosin bietet somit einen wirksamen Schutz gegenüber besonders wichtigen zellschädigenden Faktoren.

In einer bevorzugten Weiterbildung wird Phosphotyrosin zum Schutz der Haut verwendet.

Dies hat den besonderen Vorteil, daß dadurch ein Organ geschützt wird, das ständig zellschädigenden Faktoren physikalischer oder chemischer Natur, z.B. in Form der Sonnenstrahlung oder in Form von Umweltgiften, ausgesetzt ist.

In diesem Zusammenhang ist auch vorteilhaft, daß Phosphotyrosin unter oxidierenden Bedingungen, also beispielsweise an der Luft beständig ist und eine lang anhaltende Schutzwirkung z.B. gegen UV-Strahlen der Sonne ausüben kann. Es ist deshalb geeignet, als Hautschutz gegen hohe Sonneneinstrahlung eingesetzt zu werden. Der Einsatz von Phosphotyrosin zu diesem Zwecke hat ferner den Vorteil, daß es wegen seiner geringen Größe in die Haut eindringen kann und dort lange beständig ist.

Weiterhin ist es bevorzugt, Phosphotyrosin im Rahmen einer Strahlentherapie für Tumorpatienten zu verwenden.

Bei solch einer Strahlentherapie kommt es bspw. zur Anwendung ionisierender Strahlung, um maligne Neoplasien zu behandeln. Dabei ist es das Ziel, das Tumorgewebe maximal zu schädigen und gleichzeitig das umgebende gesunde Gewebe zu schonen. Aufgrund der vorstehend genannten, von den Erfindern erkannten Eigenschaften des Phosphotyrosins hat die Verwendung von Phosphotyrosin in diesem Zusammenhang den besonderen Vorteil, daß es selektiv gesundes, also Normalgewebe vor Zellschädigung schützt und für Tumorgewebe hingegen keine zytoprotektiven Eigenschaften zeigt, im Gegenteil sogar deren Absterben fördert.

Weiter ist es bevorzugt, Phosphotyrosin im Rahmen einer Chemotherapie für Tumorpatienten zu verwenden.

In der Chemotherapie werden Chemotherapeutika bzw. Zytostatika spezifisch zur Hemmung des Wachstums von Tumorzellen im Organismus eingesetzt, wobei es das Ziel ist, gesunde Zellen vor den zytotoxischen Aktivitäten der Chemotherapeutika zu schützen. Die erfindungsgemäße Verwendung des Phosphotyrosins hat somit aufgrund der erkannten und oben beschriebenen Eigenschaften den besonderen Vorteil, daß ein für gesunde Zellen selektiver Zytoprotektor bereitgestellt wird.

die Verwendung von Phosphotyrosin zur Herstellung einer pharmazeutischen Zusammensetzung zur prophylaktischen und/oder therapiebegleitenden Behandlung von Strahlentherapie- und/oder Chemotherapiepatienten, ist auch offenbart.

Dazu kann das Phosphotyrosin in den jeweils angemessenen und üblichen Galeniken zubereitet sein, d.h. gegebenenfalls neben üblichen Trägerstoffen, Hilfsmitteln und/oder Zusatzstoffen vorliegen. Solch eine pharmazeutische Zusammensetzung kann intravenös verabreicht werden, percutan, durch eine lokale Injektion bspw. in die durch den zellschädigenden Faktor direkt betroffenen Körpergebiete oder Körperhöhlen, oder auch durch lokales Auftragen.

Eine derartige Verwendung zur Herstellung einer pharmazeutischen Zusammensetzung hat außerdem den Vorteil, daß die in Rede stehende modifizierte Aminosäure als Wirksubstanz dieser Zusammensetzung seine zytoprotektive Wirkung entfalten kann, ohne gleichzeitig bedrohliche Immunreaktionen hervorzurufen. Aufgrund seiner geringen Größe hat das Phosphotyrosin nämlich nur eine niedrige Immunogenität, so daß bei Anwendung der pharmazeutischen Zusammensetzung am oder im menschlichen oder tierischen Körper keine allergische Reaktion zu erwarten ist, und daß in diesem Zusammenhang das Phosphotyrosin nicht unter Vermittlung von Antikörpern aus dem jeweiligen Organismus eliminiert wird.

Die für gesunde Normalzellen selektive zytoprotektive Aktivität und die bei Bestrahlung selektiv für Tumorzellen toxizitätsfördernde Eigenschaft des Phosphotyrosins macht dessen Verwendung zur Herstellung einer pharmazeutischen Zusammensetzung besonders geeignet.

Die Erfindung kann auch mit der Herstellung einer kosmetischen Zusammensetzung realisiert werden, die Phosphotyrosin, gegebenenfalls weitere übliche Trägerstoffe, Hilfsmittel und/oder Zusatzstoffe enthält.

Eine derartige kosmetische Zusammensetzung kann bspw. als Sonnenmilch, Hautcreme oder ähnliches dargereicht werden. Sie enthält dann die üblichen Bestandteile derartiger Zusammensetzungen wie Öle, Emulsionen, Pigmente usw. Es versteht sich, daß die kosmetische Zusammensetzung zusätzlich auch UV-Filter wie Derivate von p-amino-Benzoesäure, Salizylsäure, Zimtsäure, Dibenzoylmethan oder ähnliches enthalten kann.

Durch die zytoprotektive Wirksamkeit des Phosphotyrosins bietet eine solche kosmetische Zusammensetzung einen idealen Schutz vor allem gegen die UV-Strahlung des Sonnenlichts. Da das Phosphotyrosin aufgrund seiner geringen Größe sogar in die Haut eindringen kann und zudem lange beständig ist, kann so ein Langzeitschutz gegen Strahlung erreicht werden. Auch ist in diesem Zusammenhang an Cremes zu denken, die Personen, die verstärkten Umgang mit toxischen Chemikalien haben, einen wirksamen Hautschutz, z.B. durch Eincremen der Hände bieten.

Gegenstand der vorliegenden Erfindung ist ebenfalls ein Kultivierungsmedium, das Phosphotyrosin, gegebenenfalls weitere übliche Puffersubstanzen, Trägerstoffe, Hilfsmittel und/oder Zusatzstoffe, in einem Konzentrationsbereich von 1 bis 100 µM enthält.

Es hat sich herausgestellt, daß in Kultur befindliche Zellen oder auch Organe bei ihrem Transport, der häufig mittels Flugzeug in großer Höhe erfolgt, z.B. durch die Belastung aufgrund der Höhenstrahlung, 50 % und mehr ihrer Viabilität verlieren. Das erfindungsgemäße Kultivierungsmedium bietet durch die zytoprotektiven Eigenschaften des Phosphotyrosins dem in Kultur befindlichen biologischen Material neben geeigneten Aufbewahrungs- bzw. Kultivierungsbedingungen auch einen effektiven Schutz gegenüber zellschädigenden Faktoren, z.B. Strahlung. Damit wird gewährleistet, daß Zellproben oder Organe auch längere Transportwege, z.B. mittels Flugzeug, ohne oder mit geringem Viabilitätsrückgang überstehen.

Das erfindungsgemäß Kultivierungsmedium enthält Phosphotyrosin in einem Konzentrationsbereich von 1 bis 100 µM einzusetzen. Untersuchungen der Erfinder haben nämlich ergeben, daß in diesem Bereich die zytoprotektive Wirkung des Phosphotyrosins besonders hoch ist. Diese Erkenntnis ist vor allem deshalb überraschend, weil die oben erwähnten Experimente von Mishra und Hamburger, bei P-Tyr-Konzentrationen von 1,67 mM, zum Teil sogar von 16,7 mM durchgeführt wurden, wenngleich dort eine andere Wirkung des Phosphotyrosins gezeigt wurde.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Weitere Vorteile ergeben sich aus den folgenden Ausführungsbeispielen und im Zusammenhang mit den Zeichnungen, in denen:
- Fig. 1: die chemische Strukturformel von Phosphotyrosin (P- Tyr) bzw. O-Phospho-L-Tyrosin (L-3-[4- Hydroxyphenyl]alanin-4'-Phosphat) in nichtionisier- ter Form zeigt;
- Fig. 2: das Überleben normaler Hautfibroblasten im Vergleich zu transformierten Fibroblasten nach Vorbehandlung mit verschiedenen P-Tyr-Konzentrationen und nach ra- dioaktiver Bestrahlung zeigt;
- Fig. 3: vergleichend den radioprotektiven Effekt von Phos- photyrosin, Phosphoserin und Phosphothreonin auf normale Fibroblasten zeigt;
- Fig. 4: den radioprotektiven Effekt einer Präinkubation mit P-Tyr auf gesunde Nicht-Tumorzellen im Vergleich zu Tumorzellen zeigt;
- Fig. 5: das klonogene Überleben normaler Fibroblasten im Vergleich zu Tumorzellen nach UVB-Bestrahlung in Ab- hängigkeit einer Vorbehandlung mit P-Tyr zeigt; und
- Fig. 6: das klonogene Überleben normaler Fibroblasten im Vergleich zu Tumorzellen nach Cisplatin-Behandlung in Abhängigkeit einer Vorbehandlung mit P-Tyr zeigt.

### Beispiel 1 Für die Untersuchung der zytoprotektiven Wirkung des Phosphotyrosins verwendete Zellkulturen

Für die Untersuchung der zytoprotektiven Eigenschaften des Phosphotyrosins eignen sich besonders gut menschliche Fibroblasten (Zellstämme HSF1, HSF6, die aus menschlicher Haut stammen, Passage 9 bis 15; CCD32, der aus embryonalem Lungengewebe stammt, Passage 9 bis 11).

Zusätzlich können die menschlichen squamösen Karzinomzellinien HTB-35 (ATCC, die aus einem Cervix-Karzinom stammt, Passage unbekannt, verwendet in Passage 10 nach Erhalt) (Srivastava et al., "The status of the p53 gene in human papilloma virus positive or negative cervical carcinoma cell lines", Carcinogenesis 13, Seiten 1273-1275, 1992) und HTB-43 (ATCC, stammt aus einem hypopharyngealen Tumor, Passage 124) verwendet werden. Beide Karzinomzellinien sind durch eine Punktmutation im p53-Gen charakterisiert (Kim et al., "State of p53, Rb and DCC tumor suppressor genes in human oral cancer cell lines", Anticancer Research 13, Seiten 1405-1413, 1993).

Für die Untersuchung geeignet sind auch transformierte menschliche Fibroblasten, z.B. die Zellinie HH4dd (stammt aus menschlicher Haut, Passage 65 bis 70). Diese Zellinie leitet sich von dem normalen Zellstamm HH4 ab und ist ebenfalls durch eine p53-Mutation charakterisiert (Dittmann et al., "The radioprotective effect of BBI is associated with the activation of DNA-repair relevant genes", Int. J. Radiat. Biol. 74, Seiten 225-230, 1998). HH4dd wächst in Softagar, ist gekennzeichnet durch seine Aneuploidie und induziert Tumoren in Nacktmäusen.

Die Zellen werden in Dulbecco's Modified Eagles Medium (DMEM), versetzt mit 10%igem fötalem Kälberserum (FCS) (GIBCO/BRL, Eggenstein, Deutschland, Artikel Nr. 40G7285K) (= Normalmedium) unter Standardbedingungen kultiviert.

### Beispiel 2 Bestrahlung der Zellen, Inkubation mit Cisplatin

Die konfluent gewachsenen Zellen werden entweder in einfachem Normalmedium oder in Medium, das Phosphotyrosin, Phosphoserin oder Phosphothreonin (Sigma, München, Deutschland) in den angegebenen Konzentrationen enthält, für 16 Stunden inkubiert.

Zur radioaktiven Bestrahlung werden die Zellen mit 4MV-Photonen mittels eines "Linac"(Mevatron⁶⁰/Siemens, Erlangen, Deutschland) mit einer Dosisrate von 2 Gy/min bei Raumtemperatur wie beschrieben (Dittmann et al. "Bowman-Birk Proteinase inhibitor modulates radiosensitivity and radiation-induced differentiation of human fibroblasts in culture", Radiother. Oncol. 34, Seiten 137-143, 1995) bestrahlt.

Die Bestrahlung der Zellen mit UV-Licht (312 nm) erfolgt mit einer UVB-Lampe (Bioblock Scientific, Illkirch Cedex, Frankreich) mit einer Dosisrate von 450J/m² pro Minute.

Die Inkubation mit Cisplatin erfolgt bei einer Konzentration von 1µg/ml Cisplatin (cis-Diamminplatin(II-Dichlorid); Sigma, München, Deutschland).

### Beispiel 3 Klonogener Assay (= Koloniebildungsassay)

Die Untersuchungen zur zytoprotektiven Eigenschaft von Phosphotyrosin erfolgen im sogenannten klonogenen Assay, der bspw. von Dittmann et al., 1995, a.a.O., beschrieben ist und der im folgenden kurz erläutert wird.

Die wie in Beispiel 1 beschrieben kultivierten Zellen, die entsprechend Beispiel 2 mit den zellschädigenden Faktoren inkubiert wurden oder als Kontrolle unbehandelt bleiben, werden von Medium befreit, die Zellen werden gewaschen und mit 0,05 % Trypsin und 0,1 % EDTA vom Untergrund abgelöst. Zur Analyse der Koloniebildung werden die abgelösten Zellen in einer konstanten Zelldichte von 1.500 Zellen pro 78 cm²-Schale ausplattiert. Anschließend werden die ausplattierten Zellen in Normalmedium mit 20%igem FCS-Zusatz für 14 Tage inkubiert. Innerhalb dieser Zeit wird eine Koloniebildung ermöglicht.

Eine Kolonie ist dabei ein Zellhaufen, der innerhalb der 14- tägigen Kultivierung aus einer Einzelzelle durch aufeinanderfolgende Zellteilungen entsteht. Diese Kolonie wird auch als Klon bezeichnet. Die Anzahl an Kolonien bzw. Klone entspricht im Sinne des klonogenen Überlebens dem Ausmaß der schädigenden Wirkung eines chemischen oder physikalischen Agenz. Wenn bei der Behandlung mit den zellschädigenden Faktoren viele Zellen absterben, so bilden sich nach 14 Tagen nur wenige Kolonien aus, überleben viele Zellen, so lassen sich nach 14-tägiger Kultivierung viele Kolonien auszählen. Somit ist das klonogene Überleben der Zellen nach einer zellschädigenden Behandlung ein direktes Maß für die zytoprotektive Wirkung des Phosphotyrosins.

Nach der 14-tägigen Kultivierung werden die Zellen fixiert, gefärbt und ausgezählt, wie beschrieben (Dittmann et al., 1995, a.a.O.). Bei der Bestimmung des klonogenen Überlebens werden pro Platte die Kolonien mit mehr als 50 Zellen bestimmt. Die Auszählung erfolgt nach Kodierung der Kulturschalen und unabhängig voneinander von zwei Personen.

Das Ergebnis eines solchen klonogenen Assays wird als "relative Zytoprotektion" oder in "% Anzahl der Klone" ausgedrückt, wobei die Anzahlen der gebildeten Kolonien der verschiedenen Ansätze zueinander in Beziehung gesetzt werden.

### Beispiel 4 Reaktion der Fibroblasten auf radioaktive Bestrahlung nach Vorbehandlung mit verschiedenen Dosen an Phosphotyrosin

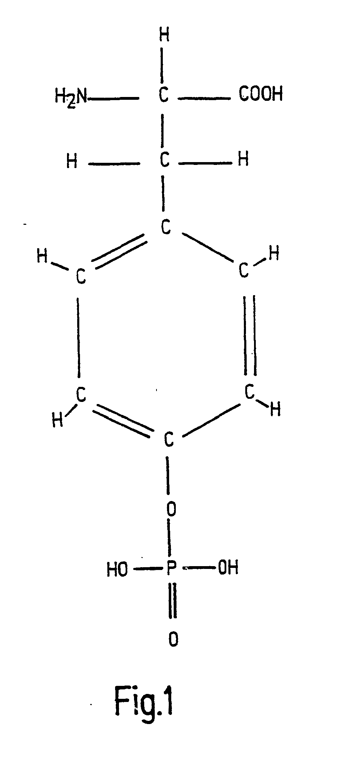

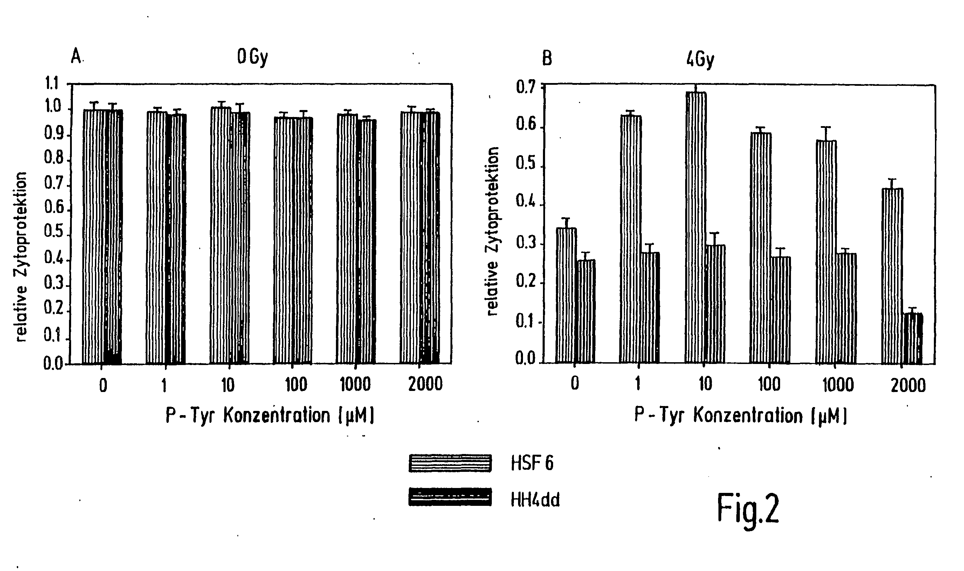

Normale Hautfibroblasten (HSF6) werden mit P-Tyr, dessen chemische Strukturformel in Fig. 1 gezeigt ist, in einem Konzentrationsbereich von 0 bis 2000 µM für 16 Stunden inkubiert. Anschließend wird ohne zellschädigende Behandlung das klonogene Überleben, wie unter Beispiel 3 angegeben, bestimmt. Das Ergebnis eines solchen Experimentes ist in Fig. 2A dargestellt.

Die hellgrauen Balken geben in dieser Abbildung die Überlebensfraktion der HSF6-Zellen an, die dunkelgrauen Balken die der HH4dd-Zellen. Dabei zeigt eine alleinige P-Tyr-Vorbehandlung bei Konzentrationen bis zu 2000 µM keinerlei Effekt auf das klonogene Überleben der HSF6-Zellen. Vergleichbare Ergebnisse werden für die transformierte Fibroblastenzellinie HH4dd erzielt. Auch hier zeigt sich, aufgrund der Ergebnisse von Mishra und Hamburger (a.a.O.) unerwarteterweise, keinerlei Auswirkung auf das klonogene Überleben (Fig. 2A).

Die kombinierte Behandlung mit Phosphotyrosin und ionisierender Bestrahlung bei einer Energiedosis von 4 Gy zeigt hingegen bei normalen Fibroblasten einen klaren Anstieg des klonogenen Überlebens. Maximales Überleben wird bei einer P-Tyr-Konzentration von 10 µM erreicht (Fig. 2B, hellgraue Balken). Bei gleicher Behandlung und gleichen Expositionsbedingungen wird bei den transformierten Fibroblasten HH4dd jedoch kein Anstieg des klonogenen Überlebens beobachtet. Ganz im Gegenteil zeigt die Behandlung der transformierten Fibroblasten mit 2000 µM P-Tyr eine signifikante Erhöhung der Strahlungstoxizität; siehe Fig. 2B.

### Beispiel 5 Vergleich des radioprotektiven Effekts von Phosphotyrosin mit Phosphoserin und Phosphothreonin

Um zu testen, ob auch andere phosphorylierte Aminosäuren, wie zum Beispiel Phosphoserin (P-Ser) oder Phosphothreonin (P-Thr) mit P-Tyr vergleichbare radioprotektive Effekte zeigen, werden nicht-transformierte Fibroblasten (HSF1) für 16 Stunden mit äquimolaren Konzentrationen dieser beiden Aminosäuren (je 10 µM) vorbehandelt, wie unter Beispiel 2 angegeben einer ionisierenden Bestrahlung mit 4 Gy unterzogen und die Ergebnisse eines klonogenen Assays mit dem des P-Tyr verglichen.

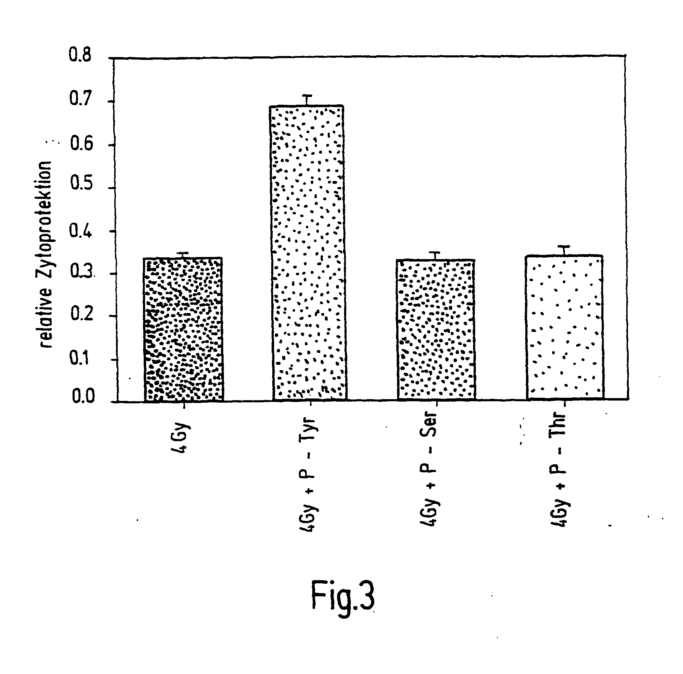

Das Ergebnis eines solchen Experimentes ist in Fig. 3 dargestellt. Es zeigt sich, daß die Präinkubation von normalen Fibroblasten mit P-Tyr zu einer signifikanten Radioprotektion führt (2. Balken von links), während die Inkubation mit P-Ser oder P-Thr unter identischen experimentellen Bedingungen zu keinerlei Radioprotektion führt (3. und 4. Balken von links).

### Beispiel 6 Reaktion von Fibroblasten auf verschiedene Dosen ionisierender Bestrahlung nach Vorbehandlung mit Phosphotyrosin

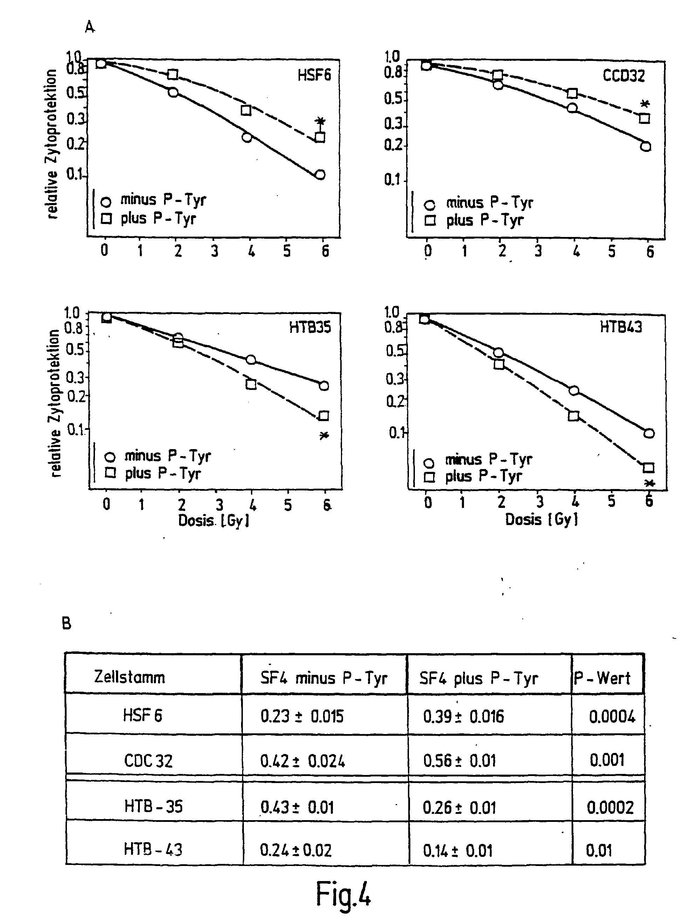

Die Zellstämme HSF6 und CCD32 sowie die Zellinien HTB-35 und HTB-43 werden mit 10 µM P-Tyr für 16 Stunden vorinkubiert. Anschließend werden die Zellen mit Dosen von 0 bis 6 Gy bestrahlt und nach einer Periode von 6 Stunden wird das klonogene Überleben bestimmt. Jeder Datenpunkt eines solchen Experimentes, dargestellt in Fig. 4A, repräsentiert den Mittelwert aus mehreren Messungen und die Standardabweichung. Die Kurvenanpassung wurde hier gemäß dem linearen quadratischen Modell berechnet, α- und β-Werte wurden bestimmt und auf Signifikanz mit Hilfe des Student t-Tests getestet. Die Sterne zeigen einen Signifikanzunterschied (p < 0,05) für α, β oder beide. In der Fig. 4B sind der Mittelwert der relativen Zytoprotektion aus den vier Meßwerten (SF4) jedes Ansatzes und die Standardabweichung einschließlich p-Wert tabellarisch dargestellt.

Die Vorbehandlung von normalen Hautfibroblasten (HSF6) und normalen Lungenfibroblasten (CCD32) mit 10 µM P-Tyr führt zu einem signifikanten Anstieg des klonogenen Überlebens bis zu einer Dosis von 6 Gy (Fig. 4A, obere Reihe, Fib. 4B Reihen 1 und 2). Hingegen resultiert die P-Tyr-Vorbehandlung der transformierten Zellinien (HTB-35, HTB-43) in einer signifikanten Reduktion des klonogenen Überlebens (Fig. 4A, untere Reihe, Fig. 4B Reihen 3 und 4).

Diese Ergebnisse zeigen, daß eine Vorbehandlung von gesunden, also Normalzellen mit Phosphotyrosin diese effektiv gegenüber zellschädigenden Faktoren schützt, hingegen eine Vorbehandlung von Tumorzellen mit Phosphotyrosin bei Inkubation mit zellschädigenden Faktoren zu einem verstärkten Absterben dieser transformierten Zellen führt.

### Beispiel 7 Reaktion von mit Phosphotyrosin vorbehandelten Fibroblasten auf UVB-Bestrahlung

Um den zytoprotektiven Schutz von Phosphotyrosin gegenüber nichtionisierender Strahlung zu testen, werden normale, nicht-transformierte Fibroblasten (HSF1) und transformierte Fibroblasten (HH4dd) für 16 Stunden mit 10 µM P-Tyr vorbehandelt, anschließend mit 200 J UVB bestrahlt und 7 Stunden später in einem klonogenen Assay, wie in Beispiel 3 beschrieben, untersucht.

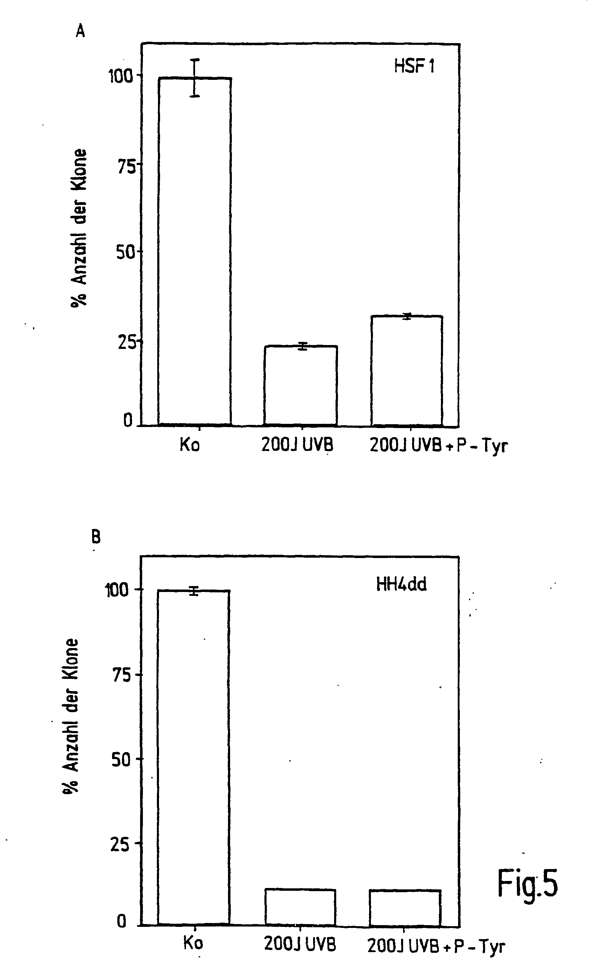

Dabei zeigt sich, daß das klonogene Überleben UVB-bestrahlter normaler nicht-transformierter Fibroblasten (HSF1) bei einer Vorbehandlung mit Phosphotyrosin um 37 % gesteigert wird (siehe Fig. 5A, Balken 2 und 3 von links).

Auf das klonogene Überleben von transformierten Fibroblasten (HH4dd) jedoch hat eine Vorbehandlung mit P-Tyr bei UVB-Bestrahlung keinerlei Auswirkungen (siehe Fig. 5B, Balken 2 und 3 von links).

Als Kontrollen (Ko) dienen in beiden Fällen unbestrahlte Zellen, wobei hier die Anzahl der gebildeten Klone 100 % darstellt.

Dieses Experiment zeigt, daß Phosphotyrosin für Nicht-Tumorzellen auch zytoprotektive Eigenschaften gegenüber nichtionisierender Strahlung hat. Auch hier ist die Zytoprotektivität selektiv für normale, nicht-transformierte Zellen. Das Überleben von transformierten Zellen nach UVB-Bestrahlung wird nämlich durch eine Vorbehandlung mit P-Tyr nicht beeinflußt.

### Beispiel 8 Reaktion von Fibroblasten auf Cisplatin-Behandlung nach Vorbehandlung mit Phosphotyrosin

Um die zytoprotektive Wirkung von Phosphotyrosin gegenüber chemischen, zellschädigenden Faktoren, zum Beispiel Zytostatika, zu untersuchen, werden normale Fibroblasten (HSF1) und transformierte Fibroblasten (HH4dd) für 16 Stunden mit 10 µM P-Tyr vorbehandelt, danach eine Stunde mit 1 µg/ml Cisplatin inkubiert. Anschließend werden die Zellen zweimal mit Normalmedium gewaschen und 6 Stunden später ausplattiert. Danach wird ein klonogener Assay, wie unter Beispiel 3 beschrieben, durchgeführt.

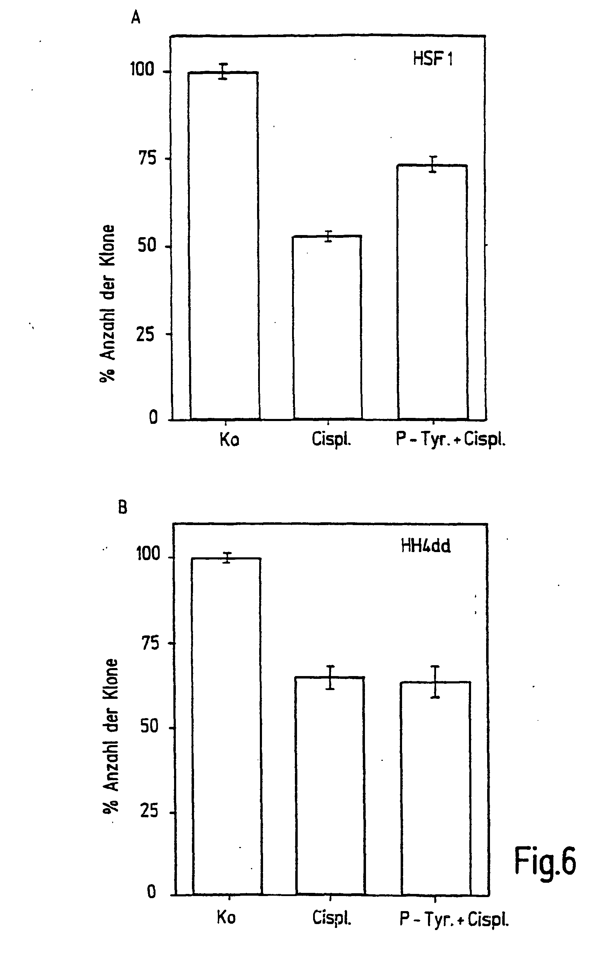

In Fig. 6 ist das Ergebnis eines solchen Experimentes dargestellt. Die Vorbehandlung von normalen, nicht-transformierten Fibroblasten (HSF1) mit P-Tyr führt im Vergleich zu nichtvorbehandelten normalen nicht-transformierten Fibroblasten (HH4dd) bei einer Cisplatininkubation zu einer Steigerung des klonogenen Überlebens von 38,7 % (siehe Fig. 6A, 2. und 3. Balken von links).

Eine P-Tyr-Vorbehandlung von transformierten Fibroblasten (HH4dd) führt bei Cisplatinbehandlung jedoch zu keiner Erhöhung des klonogenen Überlebens (Fig. 6B, 2. und 3. Balken von links).

Als Kontrollen (Ko) dienen in beiden Fällen wieder unbestrahlte Zellen, wobei hier die Anzahl der gebildeten Klone 100 % darstellt.

Folglich bietet Phosphotyrosin auch gegenüber einem zellschädigenden Faktor chemischer Art eine für gesunde, also Normalzellen selektive Zytoprotektion.

## Patentansprüche

1. Verwendung von Phosphotyrosin zur Herstellung einer pharmazeutischen Zusammensetzung zum Schutz von biologischem Material
- vor Mutagenese, Degeneration, Atrophie, Fibrosierung, Nekrose, malignem Melanom, ausgelöst durch den zellschädigenden Faktor Strahlung, vorzugsweise die ionisierende Strahlung und/oder das ultraviolette Licht,
- vor einer Regenerationshemmung der Blutzellen und Epithelien der Schleimhäute sowie der Haut und Hautanhangsgebilde, ausgelöst durch den zellschädigenden Faktor Zytostatika.

2. Verwendung nach Anspruch 1 zum Schutz der Haut.

3. Verwendung nach Anspruch 1 oder 2 im Rahmen einer Strahlentherapie für Tumorpatienten.

4. Verwendung nach einem der Ansprüche 1 bis 3 im Rahmen einer Chemotherapie für Tumorpatienten.

5. Verwendung von Phosphotyrosin zur Herstellung einer pharmazeutischen Zusammensetzung zum Schutz von gesunden Zellen
- vor Mutagenese, Degeneration, Atrophie, Fibrosierung, Nekrose, malignem Melanom, im Rahmen einer prophylaktischen und/oder therapiebegleitenden Behandlung von Strahlentherapiepatienten,
- vor einer Regenerationshemmung der Blutzellen und Epithelien der Schleimhäute sowie der Haut und Hautanhangsgebilde, im Rahmen einer prophylaktischen und/oder therapiebegleitenden Chemotherapie für Tumorpatienten.

6. Kultivierungsmedium, enthaltend Phosphotyrosin, gegebenenfalls weitere übliche Puffersubstanzen Trägerstoffe, Hilfsmittel und/oder Zusatzstoffe, **dadurch gekennzeichnet, daß** es Phosphotyrosin in einem Konzentrationsbereich von 1 bis 100 µM enthält.

## Claims

1. Use of phosphotyrosine for producing a pharmaceutical composition for protecting biological material
- from mutagenesis, degeneration, atrophy, fibrosing, necrosis, malignant melanoma, induced by the cell-damaging factor of radiation and/or ultraviolet light,
- from an inhibition of the regeneration of the blood cells and the epithelia of the mucous membranes and the skin and skin appendages, induced by the cell-damaging factor of cytostatics.

2. Use of claim 1 for the protection of the skin.

3. Use of claim 1 or 2 within the context of a radiation therapy for tumour patients.

4. Use of any of claims 1 to 3 within the context of a chemotherapy for tumour patients.

5. Use of phosphotyrosine for producing a pharmaceutical composition for protecting healthy cells
- from mutagenesis, degeneration, atrophy, fibrosing, necrosis, malignant melanoma, within the context of a prophylactic and/or therapy-accompanying treatment of radiation therapy patients,
- from an inhibition of the regeneration of the blood cells and epithelia of the mucous membranes and the skin and skin appendages, within the context of a prophylactic and/or therapy-accompanying chemotherapy for tumour patients.

6. Cultivation medium, containing phosphotyrosine and, where appropriate, other usual buffering substances, carrier substances, auxiliary substances and/or additives, **characterized in that** it contains phosphotyrosine in a concentration range of 1 to 100 µM.

## Revendications

1. Utilisation de phosphotyrosine pour la production d'une composition pharmaceutique, pour la protection de matériau biologique
- contre une mutagenèse, une dégénérescence, une atrophie, une fibrose, une nécrose, un mélanome malin déclenché par le facteur d'endommagement cellulaire qu'est le rayonnement, de préférence, le rayonnement ionique et/ou la lumière ultraviolette,
- contre une inhibition de régénérescence des cellules sanguines et épithéliales des muqueuses et de la peau ou des annexes de la peau, déclenchée par le facteur d'endommagement cellulaire constitué par les cytostatiques.

2. Utilisation selon la revendication 1, pour la protection de la peau

3. Utilisation selon la revendication 1 ou 2, dans le cadre d'une radiothérapie de patients souffrant de tumeurs.

4. Utilisation selon l'une des revendications 1 à 3, dans le cadre d'une chimiothérapie de patients souffrant de tumeurs.

5. Utilisation de phosphotyrosine pour la production d'une composition pharmaceutique pour la protection de cellules saines
- contre une mutagenèse, une dégénérescence, une atrophie, une fibrose, une nécrose, un mélanome malin dans le cadre d'un traitement prophylactique et/ou d'accompagnement thérapeutique de patients traités par rayonnement,
- contre une inhibition de régénérescence des cellules sanguines et épithéliales des muqueuses et de la peau ou des annexes de la peau, dans le cadre d'un traitement prophylactique et/ou d'accompagnement thérapeutique de patients souffrant de tumeurs.

6. Milieu de culture, contenant de la phosphotyrosine, éventuellement d'autres substances tampons, véhicules, adjuvants et/ou additifs habituels, **caractérisé en ce que** la phosphotyrosine est contenue dans une plage de concentrations de 1 à 100 µm.
